# EUROPÄISCHE PATENTSCHRIFT

(11) **EP 1 123 106 B1**
(45) Veröffentlichungstag und Bekanntmachung des Hinweises auf die Patenterteilung: **23.07.2003**
(21) Anmeldenummer: 99953897.8
(22) Anmeldetag: 20.10.1999
(51) Int. Cl.: A61K 35/78, A61P 17/02

(54) **ZUSAMMENSETZUNG ENTHALTEND EUGENOL UND POLYPHENOLE, VERWENDUNG EINER SOLCHEN ZUSAMMENSETZUNG ZUR WUNDHEILUNG**
COMPOSITION CONTAINING EUGENOL AND POLYPHENOLS AND USE OF SUCH A COMPOSITION FOR WOUND HEALING
COMPOSITION CONTENANT D'EUGENOL ET DE POLYPHENOLS ET UTILISATION D'UNE TELLE COMPOSITION POUR LA CICATRISATION

(30) Priorität: 23.10.1998 DE 19849017; 10.08.1999 DE 19937794
(43) Veröffentlichungstag der Anmeldung: 16.08.2001
(73) Patentinhaber: Ebert, Dieter, 81375 München (DE)
(72) Erfinder: Ebert, Dieter, 81375 München (DE)
(74) Vertreter: Turi, Michael, Dipl.-Phys.
(86) Internationale Anmeldenummer: EP9907967
(87) Internationale Veröffentlichungsnummer: WO00024409

(56) Entgegenhaltungen:
- EP-A- 0 060 553
- EP-A- 0 103 878
- WO-A-96/13175
- WO-A-99/59430
- STICH H. F. EL AL: "Potentiation of genotoxicity by concurrent application of compounds found in betel quid: aerecoline, eugenol, quercetin, chlorogenic acid and Mn2+" MUTATION RESEARCH, Bd. 90, 1981, Seiten 355-363, XP000892835
- DIAZ N. ET AL: "Contribucion al estudio farmacognostico de la Pulicaria paludosa Link" ANALES REAL ACADEMIA DE FARMACIA, Bd. 54, Nr. 3, 1988, Seiten 526-531, XP000892885
- SAFFORD R. J. ET AL: "Immediate contact reactions to chemicals in the fragance mix and a study of the quenching action of eugenol" BRITISH JOURNAL OF DERMATOLOGY, Bd. 123, 1990, Seiten 595-606, XP000892772

## Beschreibung

Die Erfindung betrifft eine Zusammensetzung, die aus Blättern eines Zimtbaumes gewonnen wird, zur Verwendung als Arzneimittel, ein Verfahren zur Herstellung der Zusammensetzung und die dadurch erhältliche Zusammensetzung, eine pharmazeutische Zubereitung, welche die Zusammensetzung umfaßt, die Verwendung der Zusammensetzung für die Herstellung eines Arzneimittels für die Wundheilung und ein Wundpflaster, das die Zusammensetzung enthält.

Immer mehr Menschen lehnen, falls es nicht unbedingt erforderlich ist, insbesondere bei relativ leichten Erkrankungen oder Verletzungen eine Behandlung mit synthetischen Arzneistoffen ab. Deshalb besteht ein ständiger Bedarf an Naturheilmitteln, die hochwirksame Arzneistoff-Komponenten enthalten. Insbesondere für kleinere Verletzungen wie Schnitt- und Schürfwunden, die meist nur mit einem schützenden Pflaster ohne Arzneistoff behandelt werden, wäre es wünschenswert, Zusammensetzungen auf der Basis natürlich auftretender Substanzen, die dann als Naturheilmittel zur äußerlichen Anwendung kämen, zur Verfügung zu stellen.

Der vorliegenden Erfindung liegt die Aufgabe zugrunde, natürlich auftretende Zusammensetzungen zur Verfügung zu stellen, die bspw. beschleunigend auf den Wundheilungsprozeß wirken können.

In der vorliegenden Erfindung werden nun solche Zusammensetzungen als Arzneimittel offenbart, die in Pfanzen der Gattung Cinnamomum auftretende(s) Eugenol und Polyphenole enthalten. Dabei weisen die Zusammensetzungen mindestens 60 Gew.-% Eugenol und 5 bis 20 Gew.-% Polyphenole auf. Bei den Polyphenolen handelt es sich definitionsgemäß um solche aromatischen Verbindungen, die mindestens zwei phenolische Hydroxygruppen im Molekül enthalten. Beispielhaft zu nennen wären in diesem Zusammenhang freie und/oder veretherte Polyphenole aus Blütenfarbstoffen, z.B. Anthocyanidine und/oder Flavone, oder in Gerbstoffen, z.B. Catechine oder Tannine oder jene Polyphenole, die typischerweise in Planzenblättern auftreten, insbesondere in Blättern von Zimtbäumen.

Bei Eugenol, einem weiteren Bestandteil einer erfindungsgemäßen Zusammensetzung, handelt es sich um 4-Allyl-2-methoxyphenol. Typischerweise weisen derartige erfindungsgemäße Zusammensetzungen einen sehr niedrigen Wassergehalt auf. Bevorzugt liegt der Wasseranteil in einer erfindungsgemäßen Zusammensetzung unterhalb von 5, insbesondere unterhalb von 2 Gew.-%.

In einer bevorzugten Ausführungsform der vorliegenden Erfindung enthält die Zusammensetzung von 60 bis 80 Gew.-% Eugenol und von 10 bis 20 Gew.-% Polyphenole. Ganz besonders bevorzugt ist die Zusammensetzung dann, wenn sie mindestens 0,01 Gew.-% , vorzugsweise 0,01 bis 1 Gew.-%, 1,8-Cineol, mindestens 0,1 , vorzugsweise 0,1 bis 5 Gew.-% Zimtaldehyd, 5 bis 20 Gew.-% Polyphenole und mindestens 60 Gew.-%, vorzugsweise von 60 bis 80 Gew.-%, ganz besonders bevorzugt zwischen 60 und 74 Gew.-% Eugenol enthält.

In der JP-A-1153161 (Derwent Abstract 1989-216327 [25])wird ein Trockendestillat von *Cinnamomum camphora* als deodorierendes Material offenbart. Eine medizinische Wirkung wird jedoch nicht erwähnt. Die JP-A-4368310 (Derwent Abstract 1993-041707 [05]) offenbart eine Wasserdampfdestillation von *Cinnamomoum culilauan*, deren Produkt zum Anlocken von Küchenschaben benutzt wird. Auch hier wird über eine medizinische Wirkung nichts berichtet.

Der Polyphenolbestandteil der erfindungsgemäßen Zusammensetzung stellt typischerweise eine Mischung aus verschiedenen in der Natur auftretenden Polyphenolen dar. Es kann sich aber auch um eine Einzelkomponente handeln.

Bevorzugt enthält eine erfindungsgemäße Zusammensetzung in Spuren Metallbestandteile, insbesondere Kupfer, Eisen und Lithium, wobei ein Eisenanteil in einer Konzentration von mehr als 5,000, insbesondere mehr als 10,000 ppm, bevorzugt ist.

Die erfindungsgemäße Zusammensetzung wird als Extrakt aus Blättern eines Zimtbaums (Cinnamomum), gewonnen, ganz besonders bevorzugt aus Blättern von auf Cylon und/oder den Seychellen heimischen Zimtbäumen. Bevorzugt sind die Zusammensetzungen dann, wenn sie als Extrakt aus den Blättern des Zimtbaums Cinnamomum verum J.S. Presl erhalten werden. Derartige Zusammensetzungen weisen dann typischerweise eine ölige und/oder hochviskose Konsistenz und einen gelblich bis braunen Farbton auf. Der harzähnliche Extrakt riecht intensiv nach Zimt und ist, wenn einmal auf der Haut aufgebracht, mit Wasser nicht mehr abspülbar. Zudem enthalten derartige ölige Extrakte aus Zimtbaumblättern bevorzugt mindestens 60 Gew.-% Eugenol und mindestens 10 Gew.-% Polyphenole, und darüber hinaus ggf. auch 1,8-Cineol und Zimtaldehyd im bevorzugten Konzentrationsbereich.
Der vorliegenden Erfindung liegen demnach insbesondere solche Zusammensetzungen zugrunde, die aus Blättern des Zimtbaums gewonnen werden, eine viskos-ölige Konsistenz aufweisen und zugleich die Wundheilung beschleunigen, wobei sie mindestens 60 Gew.-% Eugenol und 5 bis 20 Gew.-% Polyphenole enthalten.

Weiterhin umfaßt die vorliegende Erfindung die Verwendung einer erfindungsgemäßen Zusammensetzung, die für die Herstellung einer arzneilichen und/oder kosmetischen Zubereitung zur Wundheilung bzw. unmittelbar zur Wundheilung eingesetzt werden kann. Die erfindungsgemäße Zusammensetzung hat dabei neben einer angenehmen Geruchswirkung auch eine blutstillende und adstringierende Wirkung auf Verletzungen, wie etwa Schnitt- und/oder Schürfwunden. Zur Herstellung einer arzneilichen und/oder kosmetischen Zubereitung können weitere Hilfs- und Zusatzstoffe eingearbeitet werden, die die die Formulierung und/oder die wundheilende Wirkung der Zusammensetzung, typischerweise eines Extrakts, verbessern können.

Neben ihrer positiven Wirkung in Hinblick auf den Wundverschluß erweist sich die erfindungsgemäße Zusammensetzung aber auch als zur Desinfektion geeignet bzw. kann zur Herstellung einer arzneilichen und/oder kosmetischen Zubereitung zur Desinfektion dienen. Hierbei ist die erfindungsgemäße Zusammensetzung insbesondere im Hinblick auf deren antimikrobielle, insbesondere in Hinblick auf ihre antivirale, bakterizide und/oder fungizide Wirkung, vorteilhaft.

Als ein Verfahren zur Herstellung einer erfindungsgemäßen Zusammensetzung wird folgendes Verfahren bevorzugt: Getrocknete Blätter von Zimtbäumen, insbesondere Cinnamomum verum J.S. Presl, werden in einen Eisenkessel gegeben, der mit einem Deckel verschlossen wird. Vorzugsweise im oberen Bereich des Kessels, insbesondere am Deckel, befindet sich mindestens ein Auslaß. Der Eisenkessel wird bspw. über dem offenen Feuer erhitzt, vorzugsweise auf Temperaturen von über 100°C. Im Kessel sollte sich vorzugsweise ein Druck von mindestens 2 bar aufbauen. Sobald sich die gewünschten Druckverhältnisse eingestellt haben, wird über den Auslaß der im geschlossenen Gefäß entstehende Dampf abgelassen und über bspw. eine Kondensationsschleife kondensiert. Typischerweise wird mit der Kondensation des im Kessel entstehenden Dampfes nach mindestens 1-stündiger Erhitzung des Kessels, bevorzugt nach mindestens 3-stündiger Erhitzung, begonnen. Das derart extrahierte Kondensat mit erfindungsgemäßer Zusammensetzung kann dann z.B. in Eisengefäßen gesammelt werden.

Im Rahmen der vorliegenden Erfindung werden weiterhin Pflaster offenbart, die als Träger der einer erfindungsgemäßen Zusammensetztung oder Träger einer arzneilichen und/oder kosmetischen Zubereitung einer erfindungsgemäßen Zusammensetzung, insbesondere als Träger eines hochviskosen Öls aus den Blättern des Zimtbaums, dienen können. Es eignen sich alle im Stand der Technik gebräuchlichen Pflaster, die zur Aufnahme eines Wirkstoffs fähig sind. Ein erfindungsgemäßes Wundpflaster wird die Zusammensetzung oder arzneiliche bzw. kosmetische Zubereitung, bevorzugt aber ein öliger Extrakt aus Zimtbaumblättern, auf seiner der Wunde zugewandten Seite zur äußerlichen Anwendung so enthalten, daß die Wundheilung, insbesondere bei Schnitt- und Schürfwunden, gefördert wird.

So kann bspw. das Vlies eines Pflasters einfach mit dem Öl getränkt und dann mit einer ölabweisenden Schutzschicht bedeckt werden. Die Zusammensetzung, insbesondere als Extrakt aus Zimtbaumblättern, kann aber auch in einem Reservoir vorliegen, von wo aus es mittels leichtem Druck durch eine geeignete Membran an den Wundort geliefert wird. Weiter ist eine Verkapselung der Zusammensetzung, insbesondere des Öles aus Zimtbaumblättern, denkbar, wobei die Kapseln unmittelbar vor Gebrauch durch Druck aufgebrochen werden.

Die Erfindung wird nun anhand eines Ausführungsbeispiels weiter erläutert:

### AUSFÜHRUNGSBEISPIEL

Eine Patientin erlitt am Schienbein eine ca. 5 cm lange Schürfwunde, die sehr schmerzhaft war. Das Vlies eines langen Wundpflasterstreifens wurde mit dem oben beschriebenen, hochviskosen Öl getränkt, dann wurde das Pflaster auf die Wunde aufgebracht. Nach einer Stunde hörte die Wunde auf, weh zu tun. Bereits am nächsten Tag war die Wunde teilweise abgeheilt. Dies war umso erstaunlicher, als Wunden am Schienbein gewöhnlich schlecht heilen.

## Patentansprüche

1. Zusammensetzung, die aus Blättern eines Zimtbaumes (Cinnamomum) gewonnen wird, wobei
(a) getrocknete Blätter des Zimtbaumes in ein geschlossenes Behältnis mit mindestens einer Auslaßöffnung gegeben werden,
(b) das gemäß (a) befrachtete geschlossene Behältnis auf Temperaturen von mindestens 100°C erhitzt wird,
(c) der im Behälter entstehende Dampf über die Auslaßöffnung entweicht und nach Kondensation als Kondensat gesammelt wird,
und wobei die Zusammensetzung mindestens 60 Gew.% Eugenol und 5 bis 20 Gew.% Polyphenole enthält,
zur Verwendung als Arzneimittel.

2. Verfahren zur Herstellung einer Zusammensetzung, die aus Blättern eines Zimtbaumes gewonnen wird, wobei
(a) getrocknete Blätter des Zimtbaumes in ein geschlossenes Behältnis mit mindestens einer Auslaßöffnung gegeben werden,
(b) das gemäß (a) befrachtete geschlossene Behältnis auf Temperaturen von mindestens 100°C erhitzt wird,
(c) der im Behälter entstehende Dampf über die Auslaßöffnung entweicht und nach Kondensation als Kondensat gesammelt wird,
und wobei die Zusammensetzung mindestens 60 Gew.% Eugenol und 5 bis 20 Gew.% Polyphenole enthält,
**dadurch gekennzeichnet,**
**daß** es sich bei dem Zimtbaum um Cinnamomum verum J.S. Presl handelt.

3. Zusammensetzung, erhältlich nach dem Verfahren von Anspruch 2.

4. Pharmazeutische Zubereitung, umfassend eine Zusammensetzung nach Anspruch 1 oder 3 und mindestens ein pharmazeutisches Hilfsmittel.

5. Verwendung einer Zusammensetzung nach Anspruch 1 oder 3 für die Herstellung eines Arzneimittels für die Wundheilung.

6. Verwendung einer Zusammensetzung nach Anspruch 1 oder 3 für die Herstellung eines Arzneimittels für die Desinfektion.

7. Wundpflaster, **dadurch gekennzeichnet, daß** auf seiner der Wunde zugewandten Seite eine Zusammensetzung oder Zubereitung nach einem der Ansprüche 1, 3 und 4 aufgetragen ist.

8. Wundpflaster nach Anspruch 7, **dadurch gekennzeichnet, daß** die Zusammensetzung oder Zubereitung im Wundpflaster durch eine Membran von einem Reservoir zum Wundort permeiert.

9. Wundpflaster nach Anspruch 7 oder 8, **dadurch gekennzeichnet, daß** es die Zusammensetzung oder Zubereitung in verkapselter Form enthält.

## Claims

1. Compound, obtained from the leaves of a cinnamon tree (Cinnamomum),
(a) dried leaves of the cinnamon tree being added to a closed container with at least one outlet opening,
(b) the charged, closed container according to (a) being heated to temperatures of at least 100°C,
(c) the steam produced in the container escaping via the outlet opening and after condensation being collected as a condensate,
and the compound containing at least 60% by weight eugenol and 5 to 20% by weight polyphenols, for use as a medicament.

2. Method for the manufacture of a compound, obtained from the leaves of a cinnamon tree,
(a) dried leaves of the cinnamon tree being added to a closed container with at least one outlet opening,
(b) the charged, closed container according to (a) being heated to temperatures of at least 100°C,
(c) the steam produced in the container escaping via the outlet opening and after condensation being collected as a condensate,
and the compound containing at least 60% by weight eugenol and 5 to 20% by weight polyphenols, **characterised in that** the cinnamon tree is Cinnamomum verum J.S. Presl.

3. Compound that can be obtained according to the method of Claim 2.

4. Pharmaceutical preparation, including a compound according to Claim 1 or 3 and at least one pharmaceutical adjuvant.

5. Use of a compound according to Claim 1 or 3 for the manufacture of a medicament for healing wounds.

6. Use of a compound according to Claim 1 or 3 for the manufacture of a medicament for disinfection.

7. Wound plaster, **characterised in that** a compound or preparation according to one of Claims 1, 3 and 4 is applied to the side of it that faces the wound.

8. Wound plaster according to Claim 7, **characterised in that** the compound or preparation in the wound plaster permeates through a membrane from a reservoir to the location of the wound.

9. Wound plaster according to Claim 7 or 8, **characterised in that** it contains the compound or preparation in encapsulated form.

## Revendications

1. Composition, qui est obtenue à partir des feuilles de cannellier de Ceylan (*Cinnamomum*), où
(a) les feuilles séchées du cannellier de Ceylan sont disposées dans un récipient fermé avec au moins une ouverture d'échappement ;
(b) le récipient fermé chargé selon (a) est chauffé à une température d'au moins 100°C ;
(c) la vapeur formée dans le récipient s'échappe par l'ouverture d'échappement et après condensation, est rassemblée sous la forme d'un condensat,
et où la composition contient au moins 60% en poids d'eugénol et 5 à 20% en poids de polyphénols,
à utiliser comme agent pharmaceutique.

2. Procédé de préparation d'une composition, qui est obtenue à partir des feuilles de cannellier de Ceylan (*Cinnamomum*), où
(a) les feuilles séchées du cannellier de Ceylan sont disposées dans un récipient fermé avec au moins une ouverture d'échappement ;
(b) le récipient fermé chargé selon (a) est chauffé à une température d'au moins 100°C ;
(c) la vapeur formée dans le récipient s'échappe par l'ouverture d'échappement et après condensation, est rassemblée sous la forme d'un condensat,
et où la composition contient au moins 60% en poids d'eugénol et 5 à 20% en poids de polyphénols,
**caractérisé en ce que** le cannellier de Ceylan consiste en le *Cinnamomum verum* J.S. Presl.

3. Composition susceptible d'être obtenue selon le procédé de la revendication 2.

4. Préparation pharmaceutique, comprenant une composition selon la revendication 1 ou 3 et au moins un excipient pharmaceutique.

5. Utilisation d'une composition selon la revendication 1 ou 3, pour la préparation d'un agent pharmaceutique pour la cicatrisation.

6. Utilisation d'une composition selon la revendication 1 ou 3, pour la préparation d'un agent pharmaceutique pour la désinfection.

7. Emplâtre cicatrisant, **caractérisé en ce que** sur la face orientée vers la plaie, est appliquée une composition ou préparation selon l'une des revendications 1, 3 ou 4.

8. Emplâtre cicatrisant selon la revendication 7, **caractérisé en ce que** la composition ou préparation dans l'emplâtre cicatrisant traverse une membrane, depuis un réservoir, vers la plaie.

9. Emplâtre cicatrisant selon la revendication 7 ou 8, **caractérisé en ce qu'**il contient la composition ou préparation sous forme encapsulée.
